# EUROPEAN PATENT APPLICATION

(11) **EP 4 714 364 A1**
(43) Date of publication of application: **25.03.2026**
(21) Application number: 24825517.6
(22) Date of filing: 12.03.2024
(51) Int. Cl.: A61B 8/06

(54) **ULTRASONIC DIAGNOSTIC DEVICE, MEDICAL INFORMATION PROCESSING DEVICE, INFORMATION PROCESSING METHOD, AND PROGRAM**

(30) Priority: 20.06.2023 JP 2023100640
(71) Applicant: Canon Kabushiki Kaisha, Tokyo, 146-8501 (JP)
(72) Inventor: SASAKI, Shoya, Tokyo 146-8501 (JP); IIZUKA, Naoya, Tokyo 146-8501 (JP); SATO, Takeshi, Ohtawara-shi, Tochigi 324-8550 (JP); TAKAHASHI, Hiroki, Ohtawara-shi, Tochigi 324-8550 (JP)
(74) Representative: WESER & Kollegen Patentanwälte PartmbB
(86) International application number: PCT/JP2024/009529
(87) International publication number: WO 2024/262106

(57) **Abstract**

The present disclosure includes an ultrasonic diagnostic device including a first acquisition unit configured to acquire, based on an ultrasonic signal reflected in a living body, measurement data including tissue-derived information and blood-flow-derived information, a second acquisition unit configured to acquire blood flow data obtained by extracting or emphasizing the blood-flow-derived information of the measurement data, the blood flow data having, as a pixel value, the extracted or emphasized blood-flow-derived information, and a generation unit configured to generate, from each of the blood flow data of a plurality of frames, a plurality of pieces of feature separation data having image features different from each other, and to generate display image data by synthesizing, for the plurality of frames, feature points extracted from each of the plurality of pieces of feature separation data.

## Description

### [Technical Field]

The present invention relates to an ultrasonic diagnostic device, a medical information processing device, an information processing method, and a program.

### [Background Art]

An ultrasonic diagnostic device is widely used to observe and diagnose a blood flow in a living body. The ultrasonic diagnostic device generates and displays blood flow information from a reflected wave of an ultrasonic wave by a Doppler method based on the Doppler effect. Examples of the blood flow information generated and displayed by the ultrasonic diagnostic device include a color Doppler image, a Doppler waveform (Doppler spectrum), and the like.

The color Doppler image is captured by a color flow mapping (CFM) method. In the CFM method, ultrasonic waves are transmitted and received a plurality of times on a plurality of scanning lines. Then, by applying a moving target indicator (MTI) filter to a data string at the same position, a signal (clutter signal) derived from a stationary tissue or a slow-moving tissue is suppressed, and a signal derived from a blood flow is extracted. In the CFM method, pieces of blood flow information such as the velocity of a blood flow, the dispersion of a blood flow, and the power of a blood flow are estimated from this blood flow signal, and the distribution of an estimation result is displayed as a Doppler image.

In a B-mode image and a Doppler image, it is known that resolution deteriorates by a point spread function (PSF) determined by a wavelength of a transmission ultrasonic wave, a transmission/reception opening width, and the like. There is a solution such as increasing the frequency of the transmission ultrasonic wave, but there is also a limit to a frequency band of a probe, so that the resolution of an image that can be acquired is limited.

Non Patent Literature 1 describes a super resolution technique for a blood flow image that achieves a resolution of about 115 of a transmission ultrasonic wavelength. By extracting and integrating portions having high pixel values from a large number of blood flow images obtained in time series, a blood flow image having improved resolution is generated.

### [Citation List]

### [Non Patent Literature]

[Non Patent Literature 1] Jorgen Arendt Jensen et al., "Fast super resolution ultrasound imaging using the erythrocytes," Proc. SPIE 12038, Medical Imaging 2022: Ultrasonic Imaging and Tomography, 120380E (4 April 2022)

### [Summary of Invention]

### [Technical Problem]

In the technique disclosed in Non Patent Literature 1, for example, when blood vessels having a flow velocity difference, that is, a diameter difference are adjacent to each other and are included in an observation region, only a thick blood vessel having a fast flow velocity is extracted, and a thin blood vessel having a slow flow velocity may not appear in a blood flow image with improved resolution.

The present invention has been made in view of the above-described problems, and an object thereof is to provide a technique for obtaining high-resolution blood flow information.

### [Solution to Problem]

The present disclosure includes an ultrasonic diagnostic device including a first acquisition unit configured to acquire, based on an ultrasonic signal reflected in a living body, measurement data including tissue-derived information and blood-flow-derived information, a second acquisition unit configured to acquire blood flow data obtained by extracting or emphasizing the blood-flow-derived information of the measurement data, the blood flow data having, as a pixel value, the extracted or emphasized blood-flow-derived information, and a generation unit configured to generate, from each of the blood flow data of a plurality of frames, a plurality of pieces of feature separation data having image features different from each other, and to generate display image data by synthesizing, for the plurality of frames, feature points extracted from each of the plurality of pieces of feature separation data.

### [Advantageous Effects of Invention]

According to the present invention, high-resolution blood flow information can be obtained.

### [Brief Description of Drawings]

[Fig. 1]

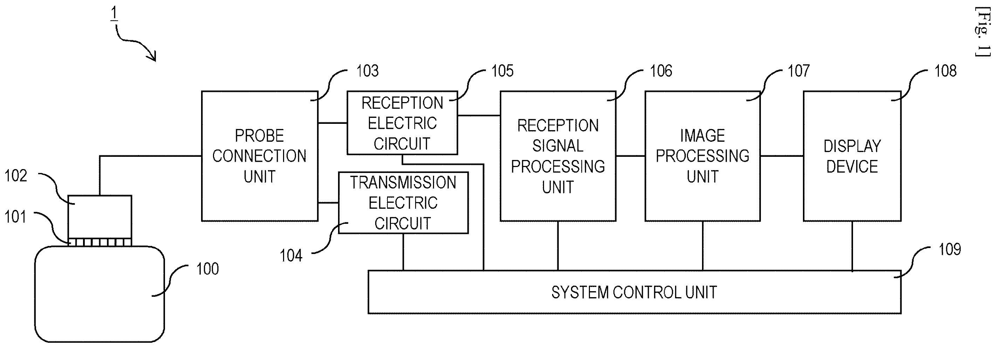

   Fig. 1 is a block diagram illustrating an example of a configuration of an ultrasonic diagnostic device.
[Fig. 2]

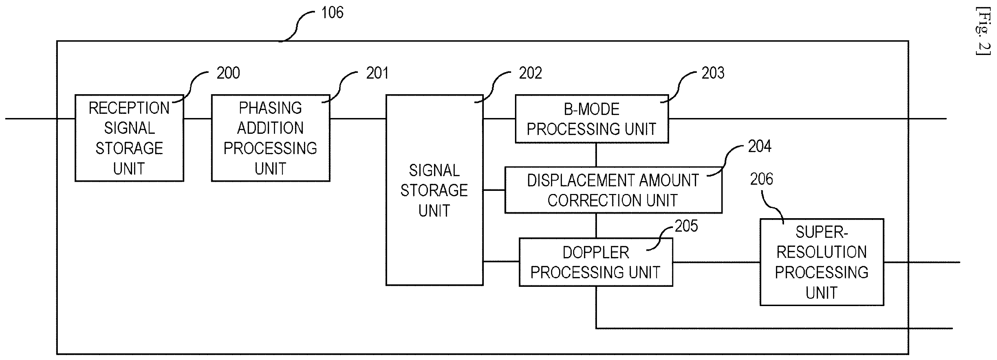

   Fig. 2 is a block diagram illustrating an example of a function of a reception signal processing block.
[Fig. 3]

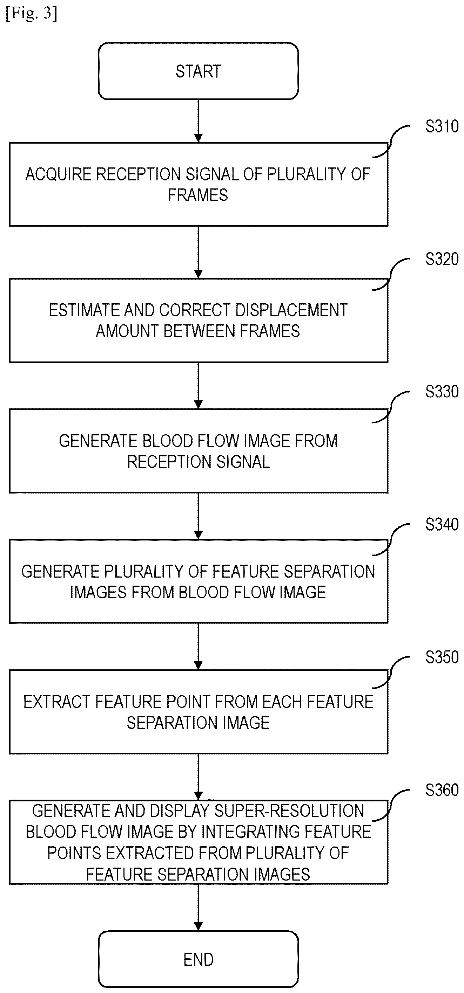

   Fig. 3 is a diagram illustrating a flow of super-resolution image generation processing according to a first embodiment.
[Fig. 4]

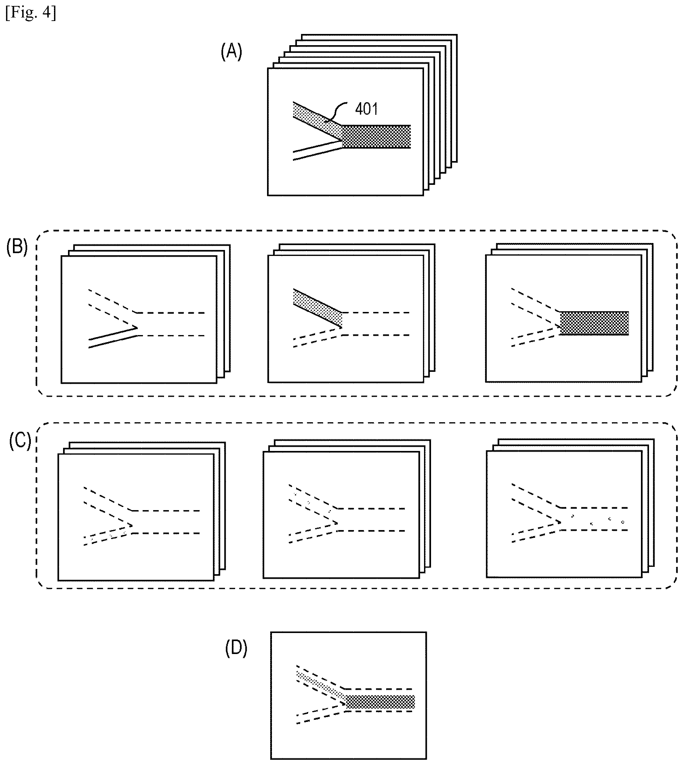

   Figs. 4A to 4D are diagrams for description of generation of a super-resolution image according to the first embodiment.
[Fig. 5]

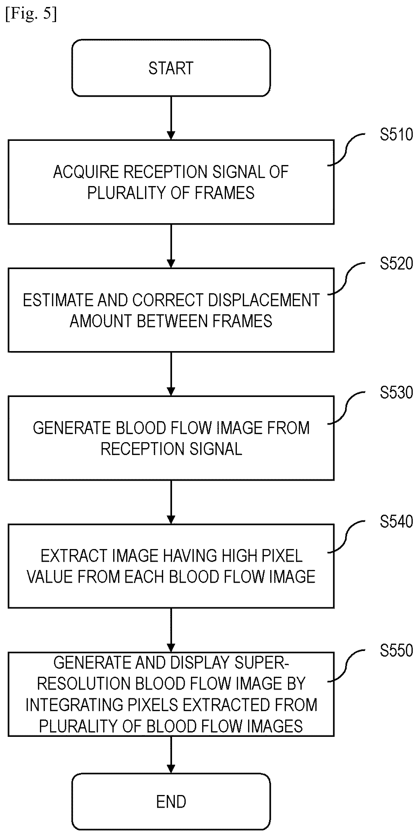

   Fig. 5 is a diagram illustrating a flow of super-resolution image generation processing of the related art.
[Fig. 6]

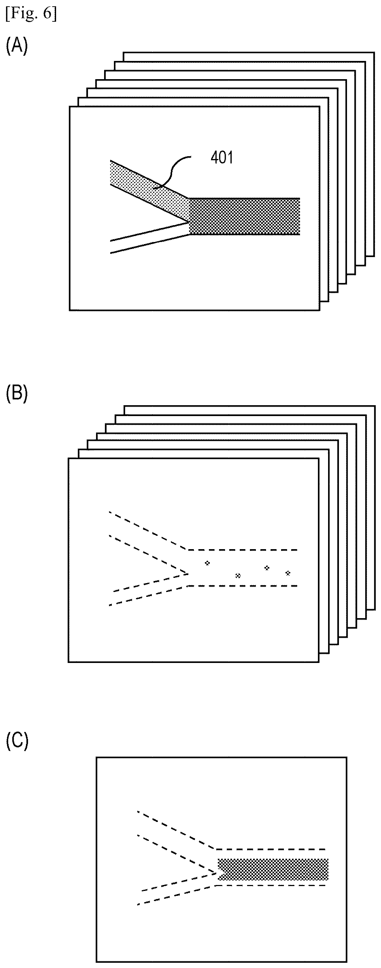

   Figs. 6A to 6C are diagrams for description of generation of a super-resolution image according to the related art.
[Fig. 7]

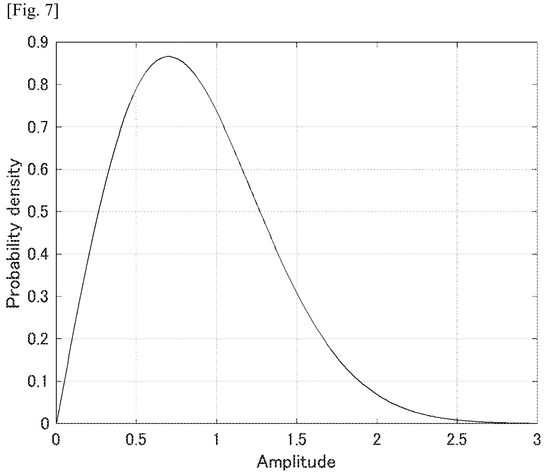

   Fig. 7 is a diagram for description of the Rayleigh distribution.
[Fig. 8]

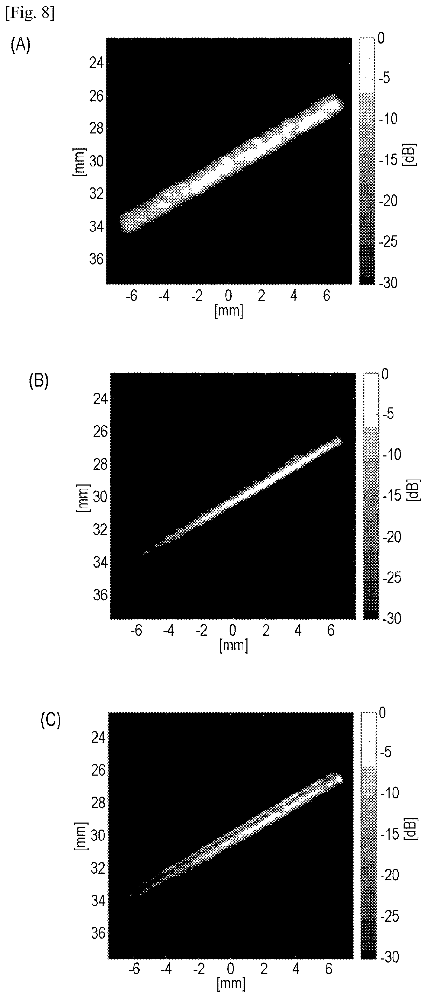

   Fig. 8A is an example of a blood flow image, Fig. 8B is an example of a super-resolution image in the related art, and Fig. 8C is an example of a super-resolution image according to the first embodiment.
[Fig. 9]

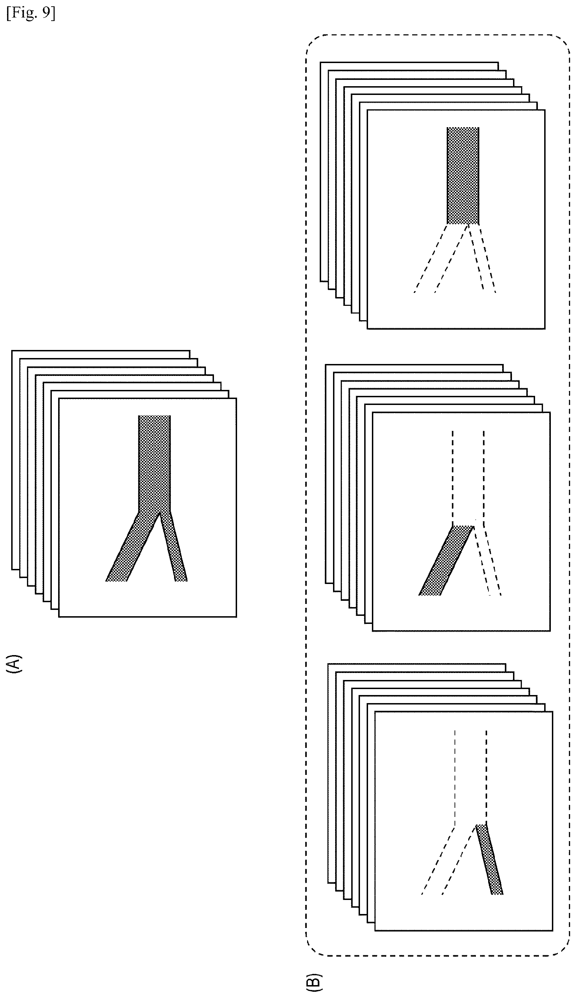

   Figs. 9A and 9B are diagrams for description of generation of a feature separation image according to a second embodiment.
[Fig. 10]

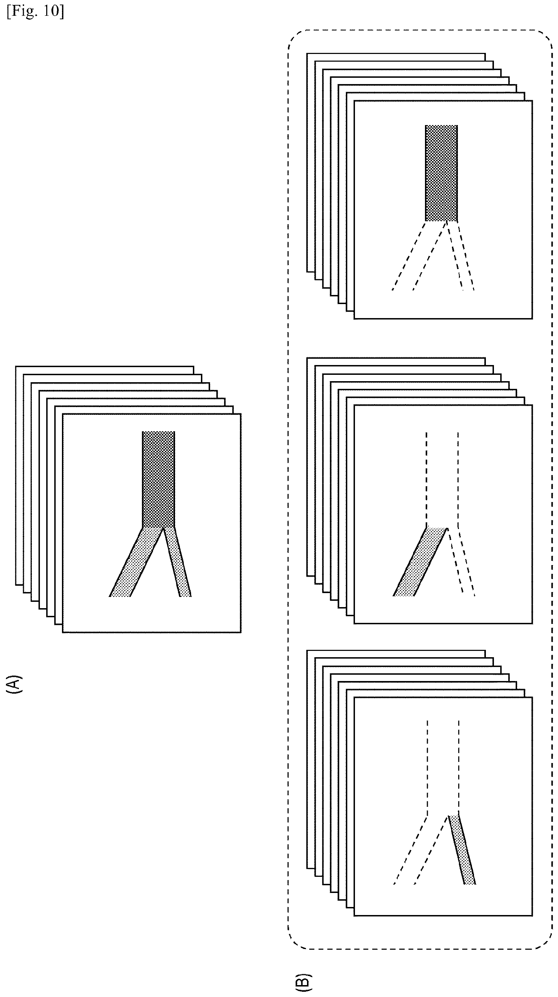

   Figs. 10A and 10B are diagrams for description of generation of a feature separation image according to a third embodiment.
[Fig. 11]

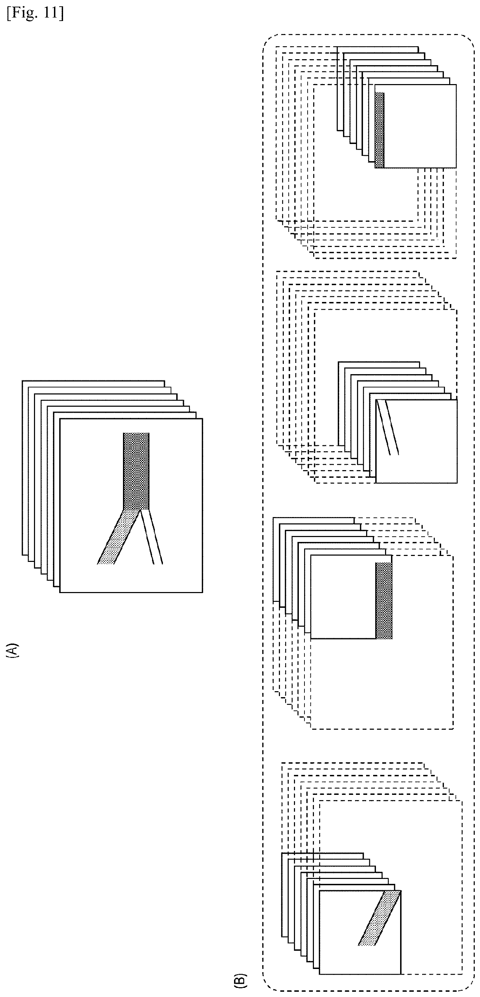

   Figs. 11A and 11B are diagrams for description of generation of a feature separation image according to a fourth embodiment.
[Fig. 12]

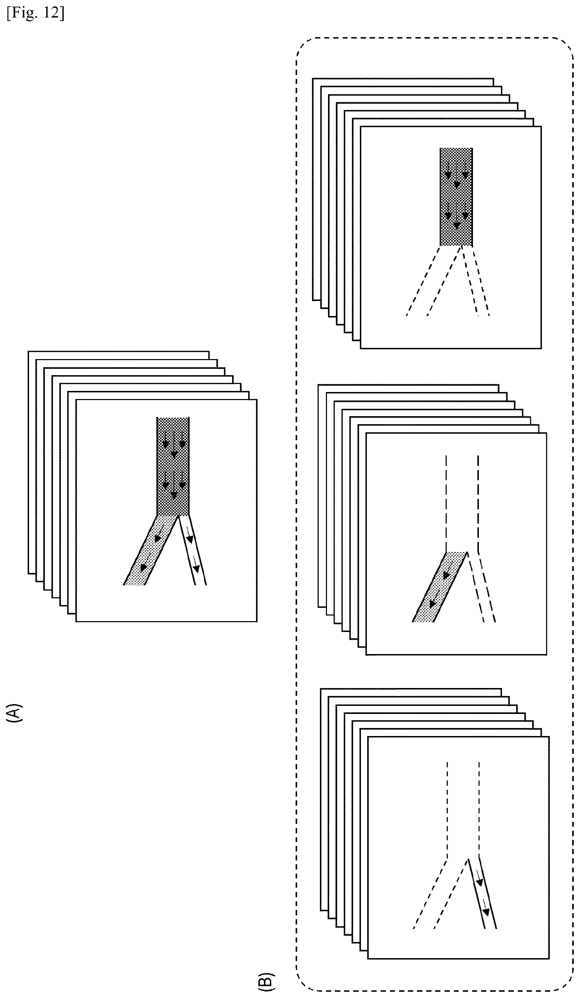

   Figs. 12A and 12B are diagrams for description of generation of a feature separation image according to a fifth embodiment.

### [Description of Embodiments]

As described above, the super-resolution technique of the related art generates a super-resolution image by a method of extracting and integrating portions each having a high pixel value from original blood flow data. Therefore, when various blood vessels are mixed in an observation region, only the pixels of blood vessels (for example, a blood vessel having a high flow velocity, a large blood vessel, and the like) each having a relatively high pixel value are used to generate the super-resolution image. As a result, in the super-resolution image, blood flow information on blood vessels (for example, a blood vessel having a slow flow velocity, a thin blood vessel, and the like) each having a relatively low pixel value may be missing.

Therefore, the present inventors adopt a new approach of generating a plurality of pieces of blood flow data (referred to as "feature separation data") having different image features from original blood flow data, and extracting pixels to be used for generating a super-resolution image from each piece of feature separation data. Specifically, the super-resolution image may be generated by the following procedure.

First, a first acquisition unit acquires, based on an ultrasonic signal reflected in a living body, measurement data including tissue-derived information (tissue component) and blood-flow-derived information (blood flow component). The "measurement data" may be, for example, a reception signal obtained by transmitting and receiving an ultrasonic signal, data obtained by performing phasing addition and orthogonal detection processing on the reception signal, and the like. Note that the blood-flow-derived information may include not only information derived from blood but also information derived from a contrast medium in blood. Time series measurement data for a plurality of frames is acquired at a predetermined frame rate.

Next, a second acquisition unit acquires blood flow data in which the blood-flow-derived information of the measurement data is extracted or emphasized. The "blood flow data" is data having the extracted or emphasized blood-flow-derived information as a pixel value, and data representing the blood flow data in a two-dimensional image format is also referred to as a "blood flow image". The "extracting blood-flow-derived information" is, for example, an operation of selectively extracting an image feature of a blood flow component from measurement data. The "emphasizing blood-flow-derived information" is, for example, an operation of making an image feature of a blood flow component relatively more prominent than an image feature of a tissue component. Note that the second acquisition unit may obtain blood flow data by performing processing of extracting or emphasizing the blood-flow-derived information, or conversely, may obtain blood flow data by performing processing of removing or reducing tissue-derived information. For example, the processing of the second acquisition unit may include processing of calculating information (also referred to as "blood flow information") such as a velocity, a dispersion value, and a power value of a blood flow by a Doppler method.

Next, a generation unit generates a plurality of pieces of feature separation data having different image features from each piece of the blood flow data of the plurality of frames. The "feature separation data" can also be said to be blood flow data including only a part of the image features (that is, a part of the blood flow information) of the original blood flow data. Data representing the feature separation data in a two-dimensional image format is also referred to as a "feature separation image". Then, the generation unit generates display image data by synthesizing feature points extracted from each of the plurality of pieces of feature separation data for a plurality of frames. The "feature point" may be a portion (that is, a portion having high probability of indicating a presence location of a blood flow) in which a feature of a blood flow is particularly strongly expressed. For example, when a feature point is extracted from an image having blood-flow-derived information (a velocity, a dispersion value, and a power value of a blood flow) as a pixel value, a pixel having a maximum pixel value in the image, a pixel having a pixel value equal to or greater than a predetermined threshold value, or the like may be extracted as the feature point. A range (extraction range) of the pixel value from which the feature point is extracted can be arbitrarily set. "Synthesis" is an operation of combining information of a plurality of feature points extracted from each of the plurality of frames to generate one blood flow image, and can be processing of, for example, integrating a plurality of feature points on one image (accumulation; plotting). The "display image data" is data obtained by imaging high-resolution blood flow information displayed as a processing result, and is also referred to as a super-resolution blood flow image.

According to the above-described procedure, it is possible to generate high-resolution display image data including more various image features than before by extracting pixels to be used in generating the display image data from each of the plurality of pieces of feature separation data having different image features, instead of directly extracting the pixels from the original blood flow data. Therefore, for example, even in a case where blood vessels having various flow velocities, diameters, or shapes are included in the observation region, it is possible to obtain a blood flow image that visualizes the blood flow of these blood vessels with high resolution.

Although an example in which the present invention is applied to the ultrasonic diagnostic device has been described so far, the present invention may be applied to a modality (a medical information processing device) other than the ultrasonic diagnostic device. For example, similarly to the ultrasonic image, it is possible to obtain high-resolution blood flow information in a medical information processing device that acquires measurement data including tissue-derived information and blood-flow-derived information from a living body.

Subsequently, some embodiments of more specific embodiments of the present invention will be exemplarily described.

### <First Embodiment>

A first embodiment of the present invention will be described. In the present embodiment, a plurality of feature separation images having different image features are generated from each of the blood flow images of a plurality of frames, feature points are extracted from each of the feature separation images, and a large number of extracted feature points are integrated to generate a super-resolution blood flow image.

A feature amount space formed by all image features included in a blood flow image is considered. For example, by separating a part of a subspace from the feature amount space and reconstructing an image only using the image features included in the subspace, it is possible to generate a feature separation image obtained by separating only a part of the image features in the original blood flow image. At this time, by making image features of interest (that is, the subspace separated from the feature amount space) different, a plurality of feature separation images having different image features can be generated from one blood flow image. Note that the subspaces of the respective feature separation images may be completely separated, or the subspaces may partially overlap each other.

Fig. 1 is a block diagram illustrating an example of a hardware configuration of an ultrasonic diagnostic device according to the present embodiment. The ultrasonic diagnostic device 1 includes an ultrasonic probe 102, a probe connection unit 103, a transmission electric circuit 104, a reception electric circuit 105, a reception signal processing unit 106, an image processing unit 107, a display device 108, and a system control unit 109. The ultrasonic diagnostic device 1 is a system for transmitting a pulsed ultrasonic signal from the ultrasonic probe 102 to an object 100, receiving the ultrasonic signal reflected in a living body, and generating image information inside the object 100. An ultrasonic image such as a B-mode image, a blood flow image, or the like obtained by the ultrasonic diagnostic device 1 is used in various clinical examinations.

The ultrasonic probe 102 is an electronic scanning probe, and has a plurality of vibrators 101 arranged one-dimensionally or two-dimensionally at the tip thereof. The vibrator 101 is an electromechanical transducer that performs mutual conversion between an electric signal (a voltage pulse signal) and an ultrasonic wave (an acoustic wave). The ultrasonic probe 102 transmits ultrasonic waves from the plurality of vibrators 101 to the object 100, and receives reflected ultrasonic waves from the object 100 by the plurality of vibrators 101. The reflected acoustic wave reflects a difference in acoustic impedance in the object 100. Note that the reflected ultrasonic signal in a case where the transmitted ultrasonic pulse is reflected by a moving blood flow, the surface of a heart wall, or the like receives frequency shift depending on a velocity component with respect to the ultrasonic transmission direction of a moving body due to the Doppler effect.

The transmission electric circuit 104 is a transmission unit that outputs a pulse signal (a drive signal) to the plurality of vibrators 101. By applying pulse signals to the plurality of vibrators 101 with a time difference, ultrasonic waves having different delay times are transmitted from the plurality of vibrators 101, thereby forming a transmission ultrasonic beam. The direction and focus of the transmission ultrasonic beam can be controlled by selectively changing the vibrator 101 to which the pulse signal is applied (that is, the vibrator 101 to be driven) or changing the delay time (application timing) of the pulse signal. An observation region inside the object 100 is scanned by sequentially changing the direction and focus of the transmission ultrasonic beam. Furthermore, by changing the delay time of the pulse signal, a transmission ultrasonic beam, which is a plane wave (focus is distant) or a diffused wave (focus point is opposite to the ultrasonic transmission direction with respect to the plurality of vibrators 101), may be formed. Alternatively, the transmission ultrasonic beam may be formed using one vibrator or a part of the plurality of vibrators 101. The transmission electric circuit 104 generates a transmission ultrasonic wave having a predetermined transmission waveform in the vibrator 101 by transmitting a pulse signal having a predetermined drive waveform to the vibrator 101. The reception electric circuit 105 is a reception unit that inputs, as a reception signal, an electric signal output from the vibrator 101 that has received the reflected ultrasonic wave. The reception signal is input to the reception signal processing unit 106.

Operation of the transmission electric circuit 104 and the reception electric circuit 105, that is, transmission and reception of ultrasonic waves is controlled by the system control unit 109. For example, the system control unit 109 changes a position where a voltage signal or a transmission ultrasonic wave is formed according to each of generation of a B-mode image and generation of a blood flow image, which will be described later.

In the case of generating a B-mode image, a reception signal of a reflected ultrasonic wave obtained by scanning an observation region is acquired and used for image generation. In the case of generating a blood flow image, ultrasonic waves are transmitted and received a plurality of times on one or a plurality of scanning lines in the observation region, and reception signals of reflected ultrasonic waves of a plurality of frames are acquired and used for image generation, that is, extraction of blood flow information. The scanning for generating the blood flow image may be performed by a method of performing transmission and reception a plurality of times on one scanning line and then performing transmission and reception on the next scanning line, or may be a method of repeating transmission and reception once on each scanning line a plurality of times. In addition, in the generation of the B-mode image and the blood flow image, the number of scanning lines may be reduced, that is, a plane wave or a diffused wave may be transmitted to transmit an ultrasonic wave over a wide range of an observation region. In addition, the transmission angle of the plane wave or the diffused wave, the range of the observation region to which the plane wave or the diffused wave is transmitted, and the like may be changed to perform transmission and reception a plurality of times in a wide range of the observation region, and the reception signals may be added and used.

In the present specification, both an analog signal output from the vibrator 101 and digital data obtained by sampling (digitally converting) the analog signal are referred to as a reception signal without particular distinction. However, for the purpose of clearly indicating that the reception signal is digital data depending on the context, the reception signal may be referred to as reception data or measurement data.

The reception signal processing unit 106 is an image generation unit that generates image data based on the reception signal obtained from the ultrasonic probe 102. The image processing unit 107 performs image processing such as luminance adjustment, interpolation, and filter processing on the image data generated by the reception signal processing unit 106. The display device 108 is a display unit for displaying image data and various types of information, and includes, for example, a liquid crystal display, an organic EL display, or the like. The system control unit 109 is a control unit that integrally controls the transmission electric circuit 104, the reception electric circuit 105, the reception signal processing unit 106, the image processing unit 107, the display device 108, and the like.

### (Configuration of Reception Signal Processing Block)

Fig. 2 is a block diagram illustrating an example of a function of the reception signal processing unit 106. The reception signal processing unit 106 includes a reception signal storage unit 200, a phasing addition processing unit 201, a signal storage unit 202, a B-mode processing unit 203, a displacement amount correction unit 204, a Doppler processing unit 205, and a super-resolution processing unit 206.

The reception signal storage unit 200 stores a reception signal received by the reception electric circuit 105. Note that, depending on a device configuration and a type of the reception signal, the reception signal may not be stored in the reception signal storage unit 200, but may be stored in the signal storage unit 202 after the phasing addition processing unit 201 to be described later. In addition, the reception signal storage unit 200 may include a block common to the signal storage unit 202 to be described later, and may store the reception signal from the reception electric circuit 105 and the reception signal after the phasing addition processing unit 201.

The phasing addition processing unit 201 performs phasing addition or orthogonal detection processing on a reception signal obtained by the reception electric circuit 105, and stores the processed reception signal in the signal storage unit 202. The phasing addition processing is processing of forming a reception ultrasonic beam by changing a delay time and a weight for each vibrator 101 and adding reception signals of a plurality of vibrators 101, and is also referred to as Delay and Sum (DAS) beamforming. The orthogonal detection processing is processing of converting a reception signal into an in-phase signal (I signal) and an orthogonal signal (Q signal) in a baseband. The phasing addition processing and the orthogonal detection processing are performed based on various conditions (opening control, signal filter) of element arrangement and image generation input from the system control unit 109. The reception signal after the phasing addition processing and the orthogonal detection processing is stored in the signal storage unit 202. Here, an example of representative DAS beamforming has been described, but any processing may be used as long as the processing forms a reception ultrasonic beam, such as adaptive beamforming, model-based processing, and processing using machine learning.

The reception signal (digital data) obtained by the reception electric circuit 105 is also referred to as RAW data. The reception signal (digital data) obtained by the phasing addition processing unit 201 is also referred to as RF data. In the present embodiment, the RAW data and the RF data are an example of measurement data acquired based on an ultrasonic signal reflected in a living body, and the reception electric circuit 105 and the phasing addition processing unit 201 are an example of a first acquisition unit that acquires measurement data.

The B-mode processing unit 203 performs envelope detection processing, logarithmic compression processing, and the like on the reception signal for generating the B-mode image stored in the signal storage unit 202, and generates image data in which signal intensity at each point in an observation region is represented by luminance intensity. In addition, the B-mode processing unit 203 may perform B-mode processing on a reception signal in which a displacement amount has been corrected by the displacement amount correction unit 204 to be described later.

The displacement amount correction unit 204 calculates a displacement amount of a tissue due to body motion or the like between the frames from the reception signals acquired from the plurality of frames. In general, the displacement amount of a tissue is calculated by block matching calculation called a speckle tracking method. A region of interest is set in the frame, and the region of interest between the frames is tracked by correlation to calculate the displacement amount of the region of interest. A displacement amount of the entire frame is calculated by setting a plurality of regions of interest in the frame. Here, an example of the method using the correlation between the frames has been described, but any method may be used as long as the displacement amount of the region of interest can be obtained. In an ultrasonic signal, speckles, which are scattered images of ultrasonic waves reflected from a scatterer in a body tissue, are tracked, and thus, this is referred to as speckle tracking. Correlation calculation of the region of interest between the frames may be performed on a reception signal after any one of the phasing addition processing, the orthogonal detection processing, and the envelope detection processing. In addition, the correlation calculation may be performed on a time waveform of the reception signal, or may be performed on frequency-domain data obtained by performing the discrete Fourier transform on the reception signal. The displacement amount correction unit 204 corrects a displacement amount by moving the reception signal with respect to a reference frame using the calculated displacement amount. The displacement amount to be corrected may be a uniform value over the entire frame, such as an average value of the displacement amounts of the respective regions of interest, or may be changed within the frame, such as for each region of interest. In addition, the displacement amount of the entire frame calculated for each region of interest may be interpolated in the time direction of a data string in the frame and the plurality of frames using linear interpolation, spline interpolation, or the like. Further, when the displacement amount is corrected, the reception signal may be similarly interpolated and then moved so as to be more finely corrected.

The Doppler processing unit 205 extracts blood flow information (Doppler information) from the reception signal for blood flow image generation, stored in the signal storage unit 202, and generates blood flow image data obtained by imaging the blood flow information. In addition, the Doppler processing unit 205 may perform the Doppler processing on the reception signal in which the displacement amount has been corrected by the above-described displacement amount correction unit 204.

A processing content of the Doppler processing unit 205 will be described in detail. The Doppler processing unit 205 extracts blood flow information based on the Doppler effect of a target object, which is located within the scanning range, by performing frequency analysis on the reception signal for blood flow image generation, which is stored in the signal storage unit 202. In the present embodiment, an example in which a target object is blood will be mainly described, but the target object may be an object such as a body tissue or a contrast medium. Examples of the blood flow information include at least one of a velocity, a dispersion value, and a power value. In addition, the Doppler processing unit 205 may obtain blood flow information at one point (one position) in the object, or may obtain blood flow information at a plurality of positions in the depth direction. In addition, the Doppler processing unit 205 may obtain the blood flow information at a plurality of time points in time series so that a temporal change of the blood flow information can be displayed.

In the generation of the blood flow image by the Doppler method, reception signal data strings of a plurality of frames are acquired in the time direction for the same measurement position. The Doppler processing unit 205 applies a moving target indicator (MTI) filter to the reception signal data string. As a result, information (clutter component) derived from a tissue that is stationary between frames or a tissue with small motion is reduced, and information (blood flow component) derived from a blood flow is extracted. Then, the Doppler processing unit 205 calculates blood flow information such as the velocity of the blood flow, the dispersion of the blood flow, and the power of the blood flow from the blood flow component. Data representing the calculated blood flow information in the form of a two-dimensional image is referred to as a blood flow image.

As the MTI filter, a filter having a fixed filter coefficient, such as a Butterworth infinite impulse response (IIR) filter or a polynomial regression filter, may be used. The MTI filter may be an adaptive filter that changes a coefficient depending on an input signal using eigenvalue decomposition, singular value decomposition, or the like. Alternatively, the Doppler processing unit 205 may decompose the reception signal into one or a plurality of bases using eigenvalue decomposition, singular value decomposition, or the like, extract only a specific basis, and remove a clutter component. In addition, the Doppler processing unit 205 may obtain a velocity vector for each coordinate in the image by using a method such as a vector Doppler method, a speckle tracking method, or a vector flow mapping method to obtain a blood flow vector representing the magnitude and the direction of a blood flow. In addition to the method exemplified here, any method may be used as long as the method is a method capable of extracting or emphasizing the blood-flow-derived information included in a reception signal (measurement data) or removing or reducing the tissue-derived information.

The super-resolution processing unit 206 generates super-resolution blood flow image data, which is a blood flow image having improved resolution, from blood flow image data for a plurality of frames generated by the Doppler processing unit 205. A method of acquiring a super-resolution blood flow image will be described in the description of a processing flow. In the present embodiment, the super-resolution processing unit 206 is an example of a generation unit that generates display image data.

The image data output from the B-mode processing unit 203, the Doppler processing unit 205, and the super-resolution processing unit 206 is processed by the image processing unit 107 illustrated in Fig. 1 and then is finally displayed on the display device 108. The respective pieces of image data may be displayed in a superimposed manner or may be displayed in parallel, or only a part of the image data may be displayed.

The reception signal processing unit 106 may include one or more processors and a memory. In this case, the function of each of the units 201 to 206 illustrated in Fig. 2 is implemented by a computer program. For example, the function of each of the units 201 to 206 can be provided by a CPU reading and executing a program stored in the memory. In addition to the CPU, the reception signal processing unit 106 may include a processor (GPU, FPGA, and the like) in charge of calculation of the B-mode processing unit 203, the displacement amount correction unit 205, the Doppler processing unit 205, and the super-resolution processing unit 206. The memory may include a memory for non-temporarily storing a program, a memory for temporarily storing data such as a reception signal, a working memory used by the CPU, and the like.

The overall configuration of the ultrasonic diagnostic device 1 according to the first embodiment has been described above. Next, a processing flow for acquiring a super-resolution blood flow image will be described.

### (Processing Flow for Obtaining Super-Resolution Blood Flow Image)

A processing flow for acquiring a super-resolution blood flow image according to the first embodiment will be described with reference to Fig. 3.

A measurement position is set so as to include a region where blood flow information is desired to be observed, and ultrasonic transmission/reception is repeatedly performed in a state of being fixed at the same measurement position to acquire a reception signal including a data string of a plurality of frames in a time direction (S310). In general, the number of frames of the reception signal when the super-resolution blood flow image according to the present embodiment is generated is larger than that when a normal Doppler image is generated. For example, the number of frames (the number of packets) of the reception signal when the normal Doppler image is generated is about 5 to 20 frames, whereas the number of frames of the reception signal when the super-resolution blood flow image is generated may be at least 100 frames or more, and preferably, may be about several hundred to several tens of thousand frames. A frame rate of the ultrasonic diagnostic device 1 is about several hundred Hz to several thousand Hz depending on a model and a measurement method. Therefore, the measurement time of the reception signal for generating the super-resolution blood flow image is about several hundred msec to several tens of sec. A reception signal including a data string of a plurality of frames is subjected to phasing addition and orthogonal detection processing by the phasing addition processing unit 201, and the reception signal is stored in the signal storage unit 202. Further, one frame in the data string may be a reception signal obtained by performing ultrasonic wave transmission/reception one time so as to include an observation region, or a frame obtained by adding a reception signal transmitted and received a plurality of times so as to include an observation region may be used as one frame. For example, a plane wave or a diffused wave is transmitted so as to include an observation region, and a plurality of reception signals, the transmission angles of which have been changed, are added to obtain data of one frame.

The displacement amount correction unit 204 calculates a displacement amount of a tissue due to body motion or the like between the frames from a reception signal including a data string of the plurality of frames. The displacement amount correction unit 204 corrects the displacement amount by moving the reception signal using the calculated displacement amount such that the positions are aligned in the frame in which integration processing in S360 or S550 to be described later is performed (S320). The reference frame at the time of calculating the displacement amount may be only one frame in the entirety of a data string, the reference frame may be changed (that is, a plurality of reference frames are provided) according to the position in the time direction, or one previous frame in the adjacent frame may be used as the reference frame. The reference frame may be arbitrarily selected from among the data strings, and for example, any one of the first frame, the intermediate frame, and the last frame may be used as a reference frame.

The Doppler processing unit 205 generates a blood flow image of a plurality of frames by applying the MTI filter to the reception signal including the data string of the plurality of frames in which the displacement amount is corrected to reduce the clutter component and extracting a component derived from the blood flow (S330). At this time, a part of the data string may be extracted and used for designing the MTI filter, or the entirety of the data string may be used. Further, parameters of the MTI filter (cutoff frequency, number of packets and number of overlaps of frames to be extracted, and the like) may be adjustable automatically or by a user depending on the properties of a reception signal, an object to be measured, and the like. Fig. 4A illustrates an example of the blood flow image of the plurality of frames, generated as described above. The density of a pixel value at each position in a position 401 including the blood flow information is caused by a difference in flow velocity. A portion having a large pixel value (a dark color in the drawing) has a fast flow velocity, and a portion having a small pixel value (a light color in the drawing) has a slow flow velocity.

Here, for comparison, a processing flow for acquiring a super-resolution blood flow image according to the related art will be described above. After the problem of the related art will be described, continuation of a processing flow for acquiring a super-resolution blood flow image according to the present embodiment will be described. Fig. 5 illustrates the processing flow for acquiring a super-resolution blood flow image according to the related art, and Figs. 6A to 6C illustrate a processing method. Steps S510 to S530 in Fig. 5 are similar to steps S310 to S330 in Fig. 3. A resulting blood flow image (Fig. 6A) of the plurality of frames is similar to that illustrated in Fig. 4A.

A pixel having a pixel value in a predetermined range is extracted from each of the blood flow images of the plurality of frames (S540). The pixel value in the predetermined range may be a maximum pixel value in the image, or may be a range of a pixel value greater than a certain threshold value. By extracting only a pixel having a pixel value in a predetermined range in each blood vessel image, an image as illustrated in Fig. 6B is obtained. A broken line in Fig. 6B represents the position 401 including blood flow information in Fig. 6A.

Since a portion having a pixel value in a predetermined range moves between frames, by integrating a plurality of blood flow images from which pixel values are extracted, a super-resolution blood flow image having improved resolution, as illustrated in Fig. 6C, is generated as compared with each blood flow image, that is, a normal Doppler image (S550). Since only a portion having a high pixel value is extracted, assuming that a point spread function is the Gaussian distribution, only a portion having a high sharpness at the center of the distribution is extracted. Although only a point or a minute range is extracted in an image of one frame, when a plurality of blood flow images from which pixel values are extracted are integrated by movement of a portion having a high pixel value between frames, a super-resolution blood flow image can be obtained.

Here, as illustrated in Figs. 6B and 6C, a problem of the related art is that, when only a pixel having a large pixel value is extracted in a blood flow image, only a pixel in a blood vessel having a large pixel value, that is, a blood vessel having a fast flow velocity is extracted.

Problems of the related art will be described in detail. In general, it is known that amplitude distribution characteristics of an ultrasonic reception signal from a uniform scattering medium can be roughly approximated by a probability distribution called the Rayleigh distribution illustrated in Fig. 7. The extraction of a portion having a high pixel value in S540 means that "a portion having a small probability but a large amplitude value in the Rayleigh distribution" is extracted from a large number of reflection signals derived from red blood cells, so that only a small number of reflection signals derived from red blood cells are spatially sparse. However, in a case where blood vessels having a difference in flow velocity are present adjacent to each other, when a portion having a high pixel value is simply extracted, only a reflection signal of "a portion having a small probability but a large amplitude value in the Rayleigh distribution of a thick blood vessel having a fast flow velocity" is extracted, and thus, a reflection signal of a thin blood vessel having a slow flow velocity is not extracted.

Fig. 8A illustrates an example of a blood flow image in a case where a "thick blood vessel having a fast flow velocity" and a "thin blood vessel having a slow flow velocity" run in parallel in the depth direction. Although the "thin blood vessel having a slow flow velocity" is arranged at a shallow position and the "thick blood vessel having a fast flow velocity" is arranged at a deep position, separation cannot be performed in the blood flow image because a blood vessel gap is equal or less than the resolution. Fig. 8B illustrates a super-resolution blood flow image according to the related art as a result of performing the processing in S540 and S550 on each blood flow image of a plurality of frames. As can be seen from comparison with Fig. 8C which illustrates a super-resolution blood flow image according to the present embodiment described later, in the super-resolution blood flow image according to the related art of Fig. 8B, it can be seen that the resolution of only a thick blood vessel having a fast flow velocity is improved, and a thin blood vessel having a slow flow velocity is not imaged.

It is also conceivable to expand an extraction range of a pixel value so that a blood vessel having a small pixel value, that is, a blood vessel having a slow flow velocity, is also extracted. However, considering extraction with respect to the Gaussian distribution described above, since the range of the extracted distribution is widened, the effect of improving resolution is reduced.

The description returns to the processing flow for acquiring the super-resolution blood flow image according to the present embodiment. In the present embodiment, in view of the above-described problems of the method of the related art, the super-resolution processing unit 206 generates a plurality of feature separation images having image features different from each other from each blood flow image (S340).

Here, an effect of dividing a blood flow image into a plurality of feature separation images will be described. Generally, in a blood flow image, between a "thick blood vessel having a high flow velocity" and a "thin blood vessel having a low flow velocity," the thick blood vessel having a high flow velocity has a larger pixel value. Therefore, when a pixel group having a pixel value in a predetermined range is extracted from a blood flow image in which a thick blood vessel having a high flow velocity and a thin blood vessel having a low flow velocity are adjacent to each other, there is a possibility that pixels of the thin blood vessel having a low flow velocity are not extracted. In order to solve this problem, a feature separation image may be generated using an image feature related to the flow velocity of a blood flow. That is, a plurality of feature separation images having different flow velocity ranges, such as a first feature separation image having an image feature corresponding to a first flow velocity range and a second feature separation image having an image feature corresponding to a second flow velocity range, are generated from an original blood flow image. Then, an appropriate extraction range is set for each feature separation image, and a feature point is extracted from each feature separation image. By such a method, both pixels of a thick blood vessel having a high flow velocity and pixels of a thin blood vessel having a low flow velocity can be extracted as feature points.

Specifically, the discrete Fourier transform is performed on a change in the pixel value of each pixel in the time direction by using two or more frames among the blood flow images of the plurality of frames. The discrete Fourier transform is a sinusoidal basis and represents a frequency component. Therefore, division can be performed by the discrete Fourier transform for each frequency component of an interframe change in each pixel of the blood flow image, that is, for each flow velocity component, and it is possible to generate a feature separation image having blood flow information in a flow velocity range corresponding to each basis. The feature separation image having the blood flow information in the flow velocity range corresponding to each basis may be an image in a frequency domain after the discrete Fourier transform, or may be an image in a time domain by performing inverse discrete Fourier transform on an image in the frequency domain. By changing a part of the frames used for the discrete Fourier transform among the blood flow images of the plurality of frames in the time direction, it is possible to obtain time-series different feature separation images corresponding to the respective basis. In this case, the blood flow image is separated into a plurality of feature separation images by focusing on image features (frequency components of temporal changes of pixel values) related to the flow velocity, that is, based on the flow velocity. Fig. 4B illustrates an example in which an image is separated into three feature separation images, and blood flows having different flow velocities are imaged in the respective bases. By increasing the number of separations, that is, the number of frames used for the discrete Fourier transform, the flow velocity range corresponding to each basis is reduced, so that the blood flow image can be separated for each finer flow velocity range. Note that, as long as the basis can be separated for each flow velocity range, a method other than the discrete Fourier transform based on a sine wave may be used, and the blood flow image may be separated into images for each flow velocity range by polynomial regression such as Legendre polynomial.

The super-resolution processing unit 206 extracts a feature point in blood-flow-derived information from the plurality of feature separation images generated in S340 (S350). The feature separation image according to the present embodiment is an image in which the blood-flow-derived information is expressed with a pixel value (luminance; concentration), and a pixel having more (stronger) blood-flow-derived information (that is, a pixel corresponding to a presence location of the blood flow) has a larger pixel value. Therefore, the super-resolution processing unit 206 extracts a point (pixel) having a large pixel value as a feature point from the feature separation image. By the operation in S350, an image in which only points where the blood-flow-derived information is particularly strongly expressed in each feature separation image are extracted as illustrated in Fig. 4C is obtained.

The super-resolution processing unit 206 may have a function as a setting unit (not illustrated) that automatically sets a pixel value range (hereinafter referred to as an "extraction range") from which a feature point is extracted or causes a user to set the pixel value range. The setting unit may read a setting value of the extraction range stored in advance in the memory and may set the setting value in the super-resolution processing unit 206. The setting unit may dynamically set the extraction range according to a pixel value distribution (histogram) or a maximum pixel value of a feature separation image. For example, the setting unit may set the maximum pixel value of the feature separation image as an upper limit, and may set a value obtained by multiplying the maximum pixel value by a predetermined coefficient (for example, 0.8 or the like) as a lower limit. At this time, if the coefficient is set to 1.0, only the pixel having the maximum pixel value in the feature separation image is extracted, and adjustment can be performed such that a larger number of pixels are extracted as the coefficient is reduced. Alternatively, an extraction range may be set so as to include the top N (N is an arbitrarily set integer) pixels of the histogram.

The super-resolution processing unit 206 may extract a pixel having the local maximum value as a feature point. For example, a blood flow image is divided into a plurality of local regions, and a feature point is extracted for each of the local regions. At this time, the super-resolution processing unit 206 may fix an extraction range of each local region, or may set an extraction range for each local region according to the local maximum pixel value. Furthermore, a pixel having a larger pixel value than an adjacent pixel (for example, when a pixel value of a central pixel becomes maximum in a local region of 3 pixels × 3 pixels), that is, a pixel having the maximum pixel value may be extracted, and the local maximum value may be extracted. Here, an example of a representative method of extracting the local maximum value has been described, but any method may be used as long as the method is processing of extracting the local maximum value. A larger number of pixels can be extracted as feature points by setting an extraction range for each local region as compared with a case in which a uniform extraction range is set for the entirety of an image, and the number of pixels to be used in the integration processing in S360 to be described later can be increased.

The setting unit may input or select the extraction range (the upper limit, the lower limit, the local region, and the like) on a setting screen displayed on the display device 108, or may designate the extraction range by a GUI such as a slider bar. At this time, the pixel value distribution (histogram) of the feature separation image may be displayed on the setting screen, or the pixel group extracted in S350 may be displayed on the setting screen. Such display can assist the user in setting the extraction range.

Alternatively, before performing the integration processing in S360, the super-resolution processing unit 206 may perform processing of actively removing pixels (noise) in the tissue from the pixel group extracted in S350. Specifically, a threshold value may be set for the pixel value distribution (histogram) of the pixel group extracted in S350, and a pixel having a pixel value smaller than the threshold value may be removed as noise. The threshold value may be set by a user. For example, the threshold value may be input or selected on a setting screen displayed on the display device 108, or may be designated by a GUI such as a slider bar. At this time, the pixel value distribution (histogram) of the pixel group extracted in S350 may be displayed on the setting screen, and further, auxiliary information such as the position of Nσ and the position of a valley of the pixel value distribution may be displayed. Such display can assist the user in selecting a threshold value. Alternatively, the super-resolution processing unit 206 may automatically set the threshold value. In this case, a fixed threshold value may be used, or the threshold value may be dynamically determined based on variance, bimodality, outlier, or the like of the pixel value distribution. Alternatively, M (M is any set integer) lower pixels may be mechanically removed as noise from a pixel group having a small pixel value among the extracted pixel groups.

In addition, the pixel value of the blood flow image varies depending on the depth from the body surface due to the influence of attenuation by a living body or diffusion attenuation of ultrasonic waves. Therefore, in the processing in S350, the super-resolution processing unit 206 may set the above-described extraction range according to the "depth from the body surface" for each local region in the feature separation image. That is, when the feature separation image includes a first local region and a second local region having different depths from the body surface, an extraction range of the first local region and an extraction range of the second local region are made different in consideration of the depth from the body surface. For example, in a case where the second local region is deeper from the body surface than the first local region and the pixel value of the second local region becomes smaller than that of the first local region due to the influence of the attenuation of the ultrasonic wave, the extraction range of the second local region may be wider (for example, the lower limit may be lowered) than the extraction range of the first local region. Alternatively, instead of changing the extraction range, processing of correcting biological attenuation or diffusion attenuation may be applied to the feature separation image before processing of extracting a feature point.

Alternatively, the super-resolution processing unit 206 may add and average the feature separation images of two or more frames to generate a feature separation image with an improved SNR, and then may extract the feature points in S350 from the added and averaged feature separation image. Further, the super-resolution processing unit 206 may not use the feature separation images of all the frames for the feature point extraction processing in S350 and the integration processing in S360. For example, a frame having a poor SNR may be excluded from processing targets in S350 and S360.

In addition, the super-resolution processing unit 206 may apply the feature point extraction processing in S350 after performing processing of increasing (up-converting) the resolution of the feature separation image. At this time, the feature separation image itself may be up-converted, or the original blood flow image may be up-converted, and the feature separation image may be generated from the blood flow image after up-conversion. Alternatively, a reception signal may be up-converted, a blood flow image may be generated using the reception signal of a plurality of frames after the up-conversion, and a feature separation image may be further generated. An up-conversion method is arbitrary, and an interpolation pixel may be generated by, for example, a nearest-neighbor, linear interpolation, spline interpolation, or the like. Alternatively, up-conversion may be performed by applying a filter generated by machine learning. Since the resolution (the number of pixels) of the super-resolution blood flow image obtained by the integration processing in S360 is the same as the resolution (the number of pixels) of an original image used to extract the feature point, the effect of improving resolving power (resolution) by the super-resolution processing is small when the resolution of the original image is low. Therefore, by increasing the resolution (the number of pixels) of the original image in advance, the resolving power of the final super-resolution blood flow image can be increased. Further, in images obtained using a convex probe or a sector probe, the pixel size (resolution) differs between a deep region and a shallow region in the image. By increasing the resolving power of the shallow region in advance by up-conversion, the effect of improving the resolving power of the final super-resolution blood flow image can also be expected.

By the extraction processing in S350, an image as illustrated in Fig. 4C is obtained. Since the processing of extracting a feature point is performed on each feature separation image for each flow velocity, it can be seen that not only a pixel of a blood vessel having a large pixel value (a blood vessel having a fast flow velocity) but also a pixel of a blood vessel having a small pixel value (a blood vessel having a slow flow velocity) can be extracted. That is, it is possible to extract a blood vessel having a flow velocity in a wider range than that in the related art. Furthermore, in a case where an energy level (for example, standard deviation of a pixel value) of a pixel value of a certain blood vessel is close to the energy of noise, the noise energy is dispersed to each basis by generating the feature separation image separated for each flow velocity by the processing in S340. As a result, the noise energy is reduced as compared with image information of a blood flow component, so that the signal-to-noise ratio (SNR) or the contrast-to-noise ratio (CNR) of each feature separation image can be improved. Therefore, there is also an advantage in that a blood vessel that cannot be distinguished from noise in the blood flow image can be depicted in the feature separation image. Furthermore, by separating an image into a plurality of images by the processing in S340, the pixel value of each image becomes sparse, and for example, in the processing in S360, a larger number of feature points can be extracted with the same number of frames than in the related art. As a result, the quality of the final super-resolution blood flow image can be improved, and the super-resolution blood flow image having the same quality can be generated from a smaller number of frames than that in the related art, so that the measurement time required to generate the super-resolution blood flow image can be shortened.

Finally, the super-resolution processing unit 206 generates a super-resolution blood flow image by integrating pixel values of the feature points extracted from a plurality of feature separation images of a large number of frames (S360). Figs. 4D and 8C illustrate examples of super-resolution blood flow images. Since pixels having a large pixel value are concentrated in a blood flow range by the integration processing, in the super-resolution blood flow image, the blood flow is imaged (visualized) with higher resolution and clarity as compared with an original blood flow image. In addition, as compared with the super-resolution blood flow image of the related art (Fig. 8B), it can be seen that both adjacent blood vessels having different thicknesses are clearly imaged in the super-resolution blood flow image (Fig. 8C) of the present embodiment. The super-resolution processing unit 206 displays the generated super-resolution blood flow image on the display device 108.

All the feature separation images generated from the blood flow images of all the frames stored in the signal storage unit 202 may be used for feature point extraction and integration processing to generate one super-resolution blood flow image, or only frames in a partial time range may be used for generation of the super-resolution blood flow image. In the latter case, a user may select a time range used for generation of the super-resolution blood flow image. In addition, a plurality of super-resolution blood flow images in time series may be generated while sequentially shifting a time range used for generation of the super-resolution blood flow image. Furthermore, moving image display of the super-resolution blood flow image may be performed by sequentially displaying a plurality of super-resolution blood flow images on the display device 108. Alternatively, a plurality of super-resolution blood flow images may be displayed side by side on the display device 108 so that temporal changes can be compared.

All the feature points extracted from the plurality of feature separation images corresponding to different image features may be simply integrated, only a part of the feature points may be selected and integrated, or the feature points may be integrated with a weight corresponding to the image features. For example, if all feature points extracted from a plurality of feature separation images having different flow velocity ranges are simply integrated, all blood vessels (blood flows) from a blood vessel having a low flow velocity to a blood vessel having a high flow velocity can be imaged. When only the feature points of a feature separation image corresponding to a specific flow velocity range are selected or the weight of the feature points is increased and then the weighted feature points are integrated, an image in which a blood vessel in the specific flow velocity range is emphasized can be obtained. A GUI for arbitrarily selecting or setting a range of image features (feature separation images) used for integration and a weight according to the image features may be provided to the user. By using such a GUI, it is possible to generate an appropriate super-resolution blood flow image according to an object to be observed (the thickness and the flow velocity of the blood vessel).

According to the configuration of the present embodiment described above, it is possible to extract a blood vessel having a wide range of flow velocity from blood flow images of a plurality of frames and to generate a super-resolution blood flow image having improved resolution.

### <Second Embodiment>

In the first embodiment, a feature separation image is generated by focusing on an image feature related to the flow velocity, but the image feature is not limited thereto. In the second embodiment, a feature separation image is generated by focusing on an image feature related to the shape or thickness of a blood vessel. That is, extracted blood vessel diameters are different in the respective bases.

For example, the super-resolution processing unit 206 performs two-dimensional Fourier transform on a spatial change in a pixel value in a blood flow image to calculate the spatial frequency of each blood flow image. A high spatial frequency, that is, a spatially fine structure, is an image feature of a thin blood vessel, and a low spatial frequency, that is, a spatially coarse structure, is an image feature of a thick blood vessel. Therefore, for example, the super-resolution processing unit 206 can generate a feature separation image having an image feature corresponding to a desired blood vessel diameter by extracting only a spatial frequency component corresponding to the desired blood vessel diameter from the two-dimensional Fourier transform of the blood flow image and performing the inverse Fourier transform. Then, by appropriately changing the spatial frequency component to be extracted, for example, a plurality of feature separation images corresponding to different blood vessel diameters, such as a feature separation image corresponding to a blood vessel diameter of 1 mm to 2 mm and a feature separation image corresponding to a blood vessel diameter of 2 mm to 3 mm, can be obtained. Fig. 9A is an example of an original blood flow image, and Fig. 9B is an example of a feature separation image of three types of blood vessel diameters generated from the original blood flow image.

In addition, by applying line emphasis processing by eigenvalue analysis of the Hessian matrix to a blood flow image, luminance and contrast of a vascular-shaped (a linear shape, a columnar shape) structure having a desired thickness are selectively emphasized, so that a feature separation image in which an image feature corresponding to a desired blood vessel diameter is emphasized may be generated. In this case as well, a plurality of feature separation images corresponding to different blood vessel diameters can be generated by appropriately changing the thickness to be emphasized by the line emphasis processing.

In addition, similarity search processing such as template matching may be used. For example, a similarity map (two-dimensional map representing similarity (correlation value) with a template image for each local region) is generated by searching the entirety of a blood flow image using a template image of a blood vessel pattern having a desired thickness. This similarity map can be regarded as a two-dimensional image filter in which a larger coefficient is set for a pixel corresponding to the blood vessel diameter of the template image. Therefore, by multiplying the blood flow image by the similarity map, a feature separation image in which an image feature corresponding to the blood vessel diameter of the template image is emphasized can be obtained. In this case, a plurality of feature separation images corresponding to different blood vessel diameters can be generated by preparing a plurality of template images having different blood vessel diameters.

Other than the method described herein, any method may be used to generate the feature separation image as long as the method selectively emphasizes or extracts the image features corresponding to the thickness and shape of the blood vessel. Note that the processing flow and the device configuration other than the feature separation image generation processing in S340 may be similar to those of the first embodiment.

### <Third Embodiment>

In a third embodiment, a feature separation image is generated based on two image features of "temporal change and spatial structure", that is, "flow velocity and shape".

For example, the super-resolution processing unit 206 performs the discrete Fourier transform (three-dimensional discrete Fourier transform) on a part of the frames among the blood flow images of the plurality of frames in both the time direction of the pixel value of each pixel and the spatial direction in the image. Then, by extracting only a spatial frequency component corresponding to a desired flow velocity range and corresponding to a desired blood vessel diameter and performing the inverse Fourier transform, it is possible to generate a feature separation image having an image feature corresponding to a desired flow velocity range and a desired blood vessel diameter. By appropriately changing the spatial frequency component to be extracted, it is possible to obtain a plurality of feature separation images having different combinations of the flow velocity range and the blood vessel diameter. Alternatively, such implementation can be performed by converting individual frames of a part of the frames among the blood flow images of the plurality of frames into a one-dimensional vector and using singular value decomposition for matrix data in which the respective frames are arranged in the column direction. As a result, images of the blood vessels having spatially different sizes can be further divided into images having different flow velocity ranges. Specifically, an image group using only each singular value component may be generated. Since the method of division into bases by the singular value decomposition is also used in the clutter removal performed in the processing in S330 of Fig. 3, the amount of operation of the entire processing can be reduced if the present processing is performed simultaneously.

As illustrated in Fig. 10A, in a case where there are blood vessels having different blood vessel diameters but the same flow velocity, the blood vessels cannot be separated at the basis of the first embodiment. On the other hand, by using the basis of the present embodiment, it is possible to further divide the blood vessels having spatially different sizes into images having different flow velocity ranges, as illustrated in Fig. 10B. As a result, the pixel value of each image becomes more sparse, and in the extraction of the feature points in the subsequent processing, a larger number of feature points can be extracted with the same number of frames than those in the related art. As a result, the quality of the final super-resolution blood flow image can be improved, and the super-resolution blood flow image having the same quality can be generated from a smaller number of frames than that in the related art, so that the measurement time required to generate the super-resolution blood flow image can be shortened.

Other than the method described herein, any method may be used to generate the feature separation image as long as the method selectively emphasizes or extracts the image features corresponding to the flow velocity and the shape of the blood vessel. Note that the processing flow and the device configuration other than the feature separation image generation processing in S340 may be similar to those of the first embodiment.

### <Fourth Embodiment>

In the fourth embodiment, a position in a blood flow image is focused as an image feature, and a feature separation image is generated. For example, the super-resolution processing unit 206 divides the blood flow image into a plurality of regions, and sets divided image (small image) for each region as a feature separation image. Fig. 11B illustrates an example in which each blood flow image in Fig. 11A is divided into four regions. By reducing a difference between pixel values of a plurality of blood vessels included in each feature separation image, a pixel of each blood vessel can be extracted.

Although the blood flow image is divided into four in Fig. 11B, the number of divisions can be arbitrarily set. In addition, the size of each region may be the same or different. Note that the processing flow and the device configuration other than the feature separation image generation processing in S340 may be similar to those of the first embodiment.

### <Fifth Embodiment>

In a fifth embodiment, a feature separation image is generated focusing on an image feature related to a direction of a blood flow.

The Doppler processing unit 205 obtains a velocity vector for each coordinate in the image by using a method such as a vector Doppler method, a speckle tracking method, or a vector flow mapping method, in addition to the blood flow image by the Doppler method. An arrow in Fig. 12A indicates the velocity vector. The direction of this velocity vector represents the direction of a blood flow. In order to generate the feature separation image corresponding to the desired blood flow direction, for example, a matching map indicating a degree of matching between a desired blood flow direction and a velocity vector for each coordinate is generated. This matching map can be regarded as a two-dimensional image filter in which a larger coefficient is set for coordinates having a velocity vector closer to a desired blood flow direction. Therefore, by multiplying the blood flow image by the matching map, a feature separation image in which an image feature corresponding to a desired blood flow direction is emphasized can be obtained. By generating the matching map for each blood flow direction and multiplying the blood flow image by the matching map, a plurality of feature separation images corresponding to different blood flow directions can be generated. Note that the generation of the matching map and the generation of the feature separation image may be performed in consideration of not only the direction of the vector but also the size of the vector.

Fig. 12B illustrates an example of a feature separation image separated for each blood flow direction (direction of the velocity vector). It can be expected that a larger number of pixels of the blood vessel can be extracted by dividing an image into images for each blood flow direction (Fig. 12B) and then extracting a feature point, rather than extracting feature points from an original blood flow image (Fig. 12A) in which various blood flow directions are mixed.

Other than the method described herein, any method may be used to generate the feature separation image as long as the method selectively emphasizes or extracts the image feature corresponding to the direction of the blood flow. Note that the processing flow and the device configuration other than the feature separation image generation processing in S340 may be similar to those of the first embodiment.

### <Other Embodiments>

The above-described embodiments merely illustrate specific examples of the present invention. The scope of the present invention is not limited to the configuration of the above-described embodiment, and various embodiments can be adopted without departing from the gist of the present invention. These embodiments and modifications thereof are included in the scope and gist of the invention and are included in the invention described in the claims and the equivalent scope thereof.

Furthermore, the disclosed technology can take an embodiment as, for example, a system, a device, a method, a program, a recording medium (storage medium), or the like. Specifically, the present invention may be applied to a system including a plurality of devices (for example, a host computer, an interface device, an imaging device, a web application, and the like), or may be applied to a device including one device.

It goes without saying that the object of the present invention is achieved by the following. In other words, a recording medium (or a storage medium) in which a program code (a computer program) of software that implements the functions of the above-described embodiments is recorded is supplied to a system or a device. Such a storage medium is, of course, a computer-readable storage medium. Then, a computer (or a CPU or an MPU) of the system or the device reads and executes the program code stored in the recording medium. In this case, a program code itself read from the recording medium implements the functions of the above-described embodiments, and the recording medium in which the program code is recorded forms the present invention.

The disclosure of the present specification includes the following configurations, a method, and a program.

### (Configuration 1)

An ultrasonic diagnostic device comprising:
a first acquisition unit configured to acquire, based on an ultrasonic signal reflected in a living body, measurement data including tissue-derived information and blood-flow-derived information;
a second acquisition unit configured to acquire blood flow data obtained by extracting or emphasizing the blood-flow-derived information of the measurement data, the blood flow data having, as a pixel value, the extracted or emphasized blood-flow-derived information; and
a generation unit configured to generate, from each of the blood flow data of a plurality of frames, a plurality of pieces of feature separation data having image features different from each other, and to generate display image data by synthesizing, for the plurality of frames, feature points extracted from each of the plurality of pieces of feature separation data.

### (Configuration 2)

The ultrasonic diagnostic device according to configuration 1, wherein
the image features include an image feature related to a flow velocity of a blood flow.

### (Configuration 3)

The ultrasonic diagnostic device according to configuration 2, wherein
the generation unit is configured to generate a feature separation image for each frequency component of a temporal change in the pixel value by performing Fourier transform on a change in a time direction of the pixel value in the blood flow data of two or more frames.

### (Configuration 4)

The ultrasonic diagnostic device according to any one of configurations 1 to 3, wherein
the image features include an image feature related to a shape of a blood vessel.

### (Configuration 5)

The ultrasonic diagnostic device according to configuration 4, wherein
the generation unit is configured to generate a feature separation image for each spatial frequency component of the blood flow data by performing Fourier transform on a spatial change in the pixel value in the blood flow data.

### (Configuration 6)

The ultrasonic diagnostic device according to any one of configurations 1 to 5, wherein
the image features include an image feature related to a flow velocity of a blood flow and a shape of a blood vessel.

### (Configuration 7)

The ultrasonic diagnostic device according to any one of configurations 1 to 6, wherein
the generation unit is configured to generate a feature separation image for each region by dividing the blood flow data into a plurality of regions.

### (Configuration 8)

The ultrasonic diagnostic device according to any one of configurations 1 to 7, wherein
the generation unit is configured to extract, as the feature point, a pixel having a pixel value in a predetermined extraction range from the feature separation data.

### (Configuration 9)

The ultrasonic diagnostic device according to configuration 8, further comprising
a setting unit configured to automatically set the extraction range or to cause a user to set the extraction range.

### (Configuration 10)

The ultrasonic diagnostic device according to configuration 9, wherein
the setting unit is configured to set the extraction range according to a maximum pixel value of the feature separation data.

### (Configuration 11)

The ultrasonic diagnostic device according to configuration 9 or 10, wherein
the setting unit is configured to: divide the feature separation data into a plurality of local regions, and set, for each of the local regions, the extraction range according to a maximum pixel value for each of the local regions.

### (Configuration 12)

The ultrasonic diagnostic device according to any one of configurations 9 to 11, wherein
the setting unit is configured to: divide the feature separation data into a plurality of local regions, and set, for each of the local regions, the extraction range according to a depth of each of the local regions from a body surface.

### (Configuration 13)

The ultrasonic diagnostic device according to any one of configurations 1 to 12, wherein
the generation unit is configured to generate the display image data by removing noise from pixel groups extracted as the feature points from the plurality of pieces of feature separation data and synthesizing the pixel groups obtained by removing the noise therefrom.

### (Configuration 14)

The ultrasonic diagnostic device according to any one of configurations 1 to 13, wherein
the generation unit is configured to extract the feature points from data obtained by averaging two or more pieces of feature separation data.

### (Configuration 15)

The ultrasonic diagnostic device according to any one of configurations 1 to 14, wherein
the generation unit is configured to: up-convert the plurality of pieces of feature separation data, and extract the feature points from the up-converted feature separation data.

### (Configuration 16)

The ultrasonic diagnostic device according to any one of configurations 1 to 15, wherein
the generation unit is configured to synthesize, when generating the display image data, the feature points extracted from the respective ones of the plurality of feature separation images by weighting the feature points according to the image features.

### (Configuration 17)

A medical information processing device comprising a generation unit configured to:
acquire blood flow data obtained by extracting or emphasizing blood-flow-derived information of measurement data including tissue-derived information and the blood-flow-derived information in a living body, the blood flow data having the extracted or emphasized blood-flow-derived information as a pixel value,
generate, from each of the blood flow data of a plurality of frames, a plurality of pieces of feature separation data having image features different from each other, and
generate display image data by synthesizing, for the plurality of frames, feature points extracted from each of the plurality of pieces of feature separation data.

### (Method 18)

An information processing method of a computer, comprising:
acquiring, based on an ultrasonic signal reflected in a living body, measurement data including tissue-derived information and blood-flow-derived information;
acquiring blood flow data obtained by extracting or emphasizing the blood-flow-derived information of the measurement data, the blood flow data having, as a pixel value, the extracted or emphasized blood-flow-derived information; and
generating, from each of the blood flow data of a plurality of frames, a plurality of pieces of feature separation data having image features different from each other, and to generate display image data by synthesizing, for the plurality of frames, feature points extracted from each of the plurality of pieces of feature separation data.

### (Program 19)

A program that causes a computer to execute an information processing method comprising:
acquiring, based on an ultrasonic signal reflected in a living body, measurement data including tissue-derived information and blood-flow-derived information;
acquiring blood flow data obtained by extracting or emphasizing the blood-flow-derived information of the measurement data, the blood flow data having, as a pixel value, the extracted or emphasized blood-flow-derived information; and
generating, from each of the blood flow data of a plurality of frames, a plurality of pieces of feature separation data having image features different from each other, and to generate display image data by synthesizing, for the plurality of frames, feature points extracted from each of the plurality of pieces of feature separation data.

The present invention is not limited to the embodiment described above, and various modifications and amendments may be implemented thereon without departing from the spirit and scope of the present invention. Accordingly, the following claims are attached in order to publicize the scope of the present invention.

The present application claims priority on the basis of Japanese Patent Application No. 2023-100640, filed on June 20, 2023, and all of the contents disclosed therein are included by reference.

### [Reference Signs List]

1: Ultrasonic diagnostic device

## Claims

1. An ultrasonic diagnostic device comprising:
a first acquisition unit configured to acquire, based on an ultrasonic signal reflected in a living body, measurement data including tissue-derived information and blood-flow-derived information;
a second acquisition unit configured to acquire blood flow data obtained by extracting or emphasizing the blood-flow-derived information of the measurement data, the blood flow data having, as a pixel value, the extracted or emphasized blood-flow-derived information; and
a generation unit configured to generate, from each of the blood flow data of a plurality of frames, a plurality of pieces of feature separation data having image features different from each other, and to generate display image data by synthesizing, for the plurality of frames, feature points extracted from each of the plurality of pieces of feature separation data.

2. The ultrasonic diagnostic device according to claim 1, wherein
the image features include an image feature related to a flow velocity of a blood flow.

3. The ultrasonic diagnostic device according to claim 2, wherein
the generation unit is configured to generate a feature separation image for each frequency component of a temporal change in the pixel value by performing Fourier transform on a change in a time direction of the pixel value in the blood flow data of two or more frames.

4. The ultrasonic diagnostic device according to claim 1, wherein
the image features include an image feature related to a shape of a blood vessel.

5. The ultrasonic diagnostic device according to claim 4, wherein
the generation unit is configured to generate a feature separation image for each spatial frequency component of the blood flow data by performing Fourier transform on a spatial change in the pixel value in the blood flow data.

6. The ultrasonic diagnostic device according to claim 1, wherein
the image features include an image feature related to a flow velocity of a blood flow and a shape of a blood vessel.

7. The ultrasonic diagnostic device according to claim 1, wherein
the generation unit is configured to generate a feature separation image for each region by dividing the blood flow data into a plurality of regions.

8. The ultrasonic diagnostic device according to claim 1, wherein
the generation unit is configured to extract, as the feature point, a pixel having a pixel value in a predetermined extraction range from the feature separation data.

9. The ultrasonic diagnostic device according to claim 8, further comprising
a setting unit configured to automatically set the extraction range or to cause a user to set the extraction range.

10. The ultrasonic diagnostic device according to claim 9, wherein
the setting unit is configured to set the extraction range according to a maximum pixel value of the feature separation data.

11. The ultrasonic diagnostic device according to claim 9, wherein
the setting unit is configured to: divide the feature separation data into a plurality of local regions, and set, for each of the local regions, the extraction range according to a maximum pixel value for each of the local regions.

12. The ultrasonic diagnostic device according to claim 9, wherein
the setting unit is configured to: divide the feature separation data into a plurality of local regions, and set, for each of the local regions, the extraction range according to a depth of each of the local regions from a body surface.

13. The ultrasonic diagnostic device according to claim 1, wherein
the generation unit is configured to generate the display image data by removing noise from pixel groups extracted as the feature points from the plurality of pieces of feature separation data and synthesizing the pixel groups obtained by removing the noise therefrom.

14. The ultrasonic diagnostic device according to claim 1, wherein
the generation unit is configured to extract the feature points from data obtained by averaging two or more pieces of feature separation data.

15. The ultrasonic diagnostic device according to claim 1, wherein
the generation unit is configured to: up-convert the plurality of pieces of feature separation data, and extract the feature points from the up-converted feature separation data.

16. The ultrasonic diagnostic device according to claim 1, wherein
the generation unit is configured to synthesize, when generating the display image data, the feature points extracted from the respective ones of the plurality of feature separation images by weighting the feature points according to the image features.

17. A medical information processing device comprising a generation unit configured to:
acquire blood flow data obtained by extracting or emphasizing blood-flow-derived information of measurement data including tissue-derived information and the blood-flow-derived information in a living body, the blood flow data having the extracted or emphasized blood-flow-derived information as a pixel value,
generate, from each of the blood flow data of a plurality of frames, a plurality of pieces of feature separation data having image features different from each other, and
generate display image data by synthesizing, for the plurality of frames, feature points extracted from each of the plurality of pieces of feature separation data.

18. An information processing method of a computer, comprising:
acquiring, based on an ultrasonic signal reflected in a living body, measurement data including tissue-derived information and blood-flow-derived information;
acquiring blood flow data obtained by extracting or emphasizing the blood-flow-derived information of the measurement data, the blood flow data having, as a pixel value, the extracted or emphasized blood-flow-derived information; and
generating display image data by generating, from each of the blood flow data of a plurality of frames, a plurality of pieces of feature separation data having image features different from each other, and synthesizing, for the plurality of frames, feature points extracted from each of the plurality of pieces of feature separation data.

19. A program that causes a computer to execute an information processing method comprising:
acquiring, based on an ultrasonic signal reflected in a living body, measurement data including tissue-derived information and blood-flow-derived information;
acquiring blood flow data obtained by extracting or emphasizing the blood-flow-derived information of the measurement data, the blood flow data having, as a pixel value, the extracted or emphasized blood-flow-derived information; and
generating display image data by generating, from each of the blood flow data of a plurality of frames, a plurality of pieces of feature separation data having image features different from each other, and synthesizing, for the plurality of frames, feature points extracted from each of the plurality of pieces of feature separation data.
